# EUROPEAN PATENT SPECIFICATION

(11) **EP 3 625 224 B1**
(45) Date of publication and mention of the grant of the patent: **04.08.2021**
(21) Application number: 18725223.4
(22) Date of filing: 17.05.2018
(51) Int. Cl.: C07D 403/12, A61P 35/00, A61K 31/675

(54) **N-SUBSTITUTED INDOLE DERIVATIVES**
N-SUBSTITUIERTE INDOLDERIVATE
DÉRIVÉS D'INDOLE N-SUBSTITUÉS

(30) Priority: 18.05.2017 WO PCT/EP2017/062008
(43) Date of publication of application: 25.03.2020
(73) Proprietor: Idorsia Pharmaceuticals Ltd, 4123 Allschwil (CH)
(72) Inventor: FRETZ, Heinz, 3654 Gunten (CH); LYOTHIER, Isabelle, 4123 Allschwil (CH); POTHIER, Julien, 4123Allschwil (CH); RICHARD-BILDSTEIN, Sylvia, 4123 Allschwil (CH); SIFFERLEN, Thierry, 4123 Allschwil (CH)
(74) Representative: Velker, Jörg
(86) International application number: PCT/EP2018/062865
(87) International publication number: WO 2018/210995

(56) References cited:
- EP-A1- 1 661 896
- EP-A1- 2 014 657
- WO-A1-2012/127032

## Description

The present invention relates to novel N-substituted indole derivatives of formula (I) and their use as pharmaceuticals. The invention also concerns related aspects including processes for the preparation of the compounds, pharmaceutical compositions containing one or more compounds of formula (I), and their use as modulators of the PGE2 receptor EP2 (alias PTGER2, alias PGE2 Receptor EP2 Subtype). The compounds of formula (I) may especially be used as single agents or especially in combination with one or more therapeutic agents such as especially a modulator of the PGE2 receptor EP4 (alias PTGER4, alias EP4R, alias PGE2 Receptor EP4 Subtype); and/or chemotherapy and/or radiotherapy and/or immunotherapy in the prevention/prophylaxis or treatment of cancers; in particular the prevention/prophylaxis or treatment of melanoma; lung cancer; bladder cancer; renal carcinomas; gastro-intestinal cancers; endometrial cancer; ovarian cancer; cervical cancer; and neuroblastoma.

Prostaglandin E2 (PGE2) is a bioactive lipid that can elicit a wide range of biological effects associated with inflammation and cancer. PGE2 belongs to the prostanoid family of lipids. Cyclooxygenase (COX) is the rate-limiting enzyme in the synthesis of biological mediators termed prostanoids, consisting of prostaglandin PGD2, PGE2, PGF2a, prostacyclin PGI2, and thromboxane TXA2. Prostanoids function via activation of seven transmembrane G-protein-coupled receptors (GPCRs), in particular EP1, EP2, EP3, and EP4 are receptors for PGE2. Activation of both EP2 and EP4 by PGE2 stimulates adenylate cyclase, resulting in elevation of cytoplasmic cAMP levels to initiate multiple downstream events via its prototypical effector Protein kinase A. In addition, PGE2 is also able to signal via PI3K/AKT and Ras-MAPK/ERK signalling

Cancers figure among the leading causes of death worldwide. Tumors are comprised of abnormally proliferating malignant cancer cells but also of a functionally supportive microenvironment. This tumor microenvironment is comprised of a complex array of cells, extracellular matrix components, and signaling molecules and is established by the altered communication between stromal and tumor cells. As tumors expand in size, they elicit the production of diverse factors that can help the tumor to grow such as angiogenic factors (promoting ingrowth of blood vessels) or that can help to evade the attack of the host immune response. PGE2 is such an immunomodulatory factor produced in tumors.

It is well established that COX2, mainly via PGE2, promotes overall growth of tumors and is upregulated and correlates with clinical outcome in a high percentage of common cancers, especially colorectal, gastric, esophageal, pancreatic, breast and ovarian cancer. High COX-2 and PGE2 expression levels are associated with neoplastic transformation, cell growth, angiogenesis, invasiveness, metastasis and immune evasion.

The finding that COX2 is over-expressed and plays an important role in carcinogenesis in gastrointestinal (GI) cancers including among others esophagus, gastric and colorectal cancers has led to the fact that COX-inhibitors (Coxibs), including Celecoxib, and other nonsteroidal anti-inflammatory drugs (NSAID), including aspirin, are among the most studied cancer chemopreventive agents in development today (for review see for example Wang R et al, Curr Pharm Des. 2013;19(1):115-25; Garcia Rodriguez LA et al, Recent Results Cancer Res. 2013;191:67-93, Sahin IH et al, Cancer Lett. 2014 Apr 10;345(2):249-57; Drew DA et al, Nat Rev Cancer 2016, 16:173; Brotons C et al, Am J Cardiovasc Drugs. 2015 Apr; 15(2):113)

In addition to COX2 and PGE2, also EP receptors, especially EP2 and EP4, are aberrantly over-expressed in multiple types of cancers, especially in gastro-intestinal (GI) cancers and pancreatic cancer. Furthermore, the over-expression of PGE2 and/or EP2 and/or EP4 correlates with diseases progression in some cancer types such as oesophageal squamous cell carcinoma (Kuo KT et al, Ann Surg Onc 2009; 16(2), 352-60); squamous cell carcinoma of the lung (Alaa M et al, Int J Oncol 2009, 34(3); 805-12); prostate cancer (Miyata Y et al, Urology 2013, 81(1):136-42): Badawi AF and Badr MZ Int J Cancer. 2003, 103(1):84-90); head and neck squamous cell carcinoma (Gallo O et al, Hum Pathol. 2002, 33(7):708-14).

In accordance to studies performed with Coxibs, in mice, knockout of either COX1, COX2, microsomal prostaglandin E synthase 1 (mPTGES1), EP2 or EP4 resulted in reduced tumor incidence and progression in different tumor models. Conversely, overexpression of COX2 or mPTGES1 in transgenic mice resulted in increased tumor incidence and tumor burden (for review see Nakanishi M. and Rosenberg D.W., Seminars in Immunopathology 2013, 35: 123-137; Fischer SM et al Cancer Prev Res (Phila) 2011 Nov;4(11):1728-35; Fulton AM et al Cancer Res 2006; 66(20); 9794-97).

Several pharmacological studies to inhibit tumor growth and progression using EP receptor antagonists or COX2 inhibitors in different tumor models have been conducted in mice. Among others, EP antagonists and/or COX2 inhibitors reduced tumor growth and metastasis in experimental models of colorectal cancer (e.g Yang L et al Cancer Res 2006, 66(19), 9665-9672; Pozzi A.et al JBC 279(28); 29797-29804), lung carcinomas (Sharma S et al Cancer Res 2005 65(12), 5211-5220), gastro-intestinal cancer (Oshima H et al Gastroenterology 2011, 140(2); 596-607; Fu SL et al world J Gastroenterol 2004, 10(13); 1971-1974), breast cancer (Kundu N et al, Breast Cancer Res Treat 117, 2009; 235-242; Ma X et al, Oncolmmunology 2013; Xin X et al Lab Investigation 2012, 1-14; Markosyan N et al; Breast Cancer Res 2013, 15:R75), prostate cancer (Xu S et al, Cell Biochem Biophys 2014, Terada et al Cancer Res 70(4) 2010; 1606-1615), pancreatic cancer (Al-Wadei HA et al, PLOS One 2012, 7(8):e43376; Funahashi H et al, Cancer Res 2007, 67(15):7068-71). COX2 inhibitors were approved for the treatment of familial adenomatous polyposis (FAP) which is an inherited pre-disposition syndrome for colorectal cancer, but later retracted due to cardiovascular side effects.

Mechanistically, PGE2 signalling is mainly involved in the crosstalk between tumor and stromal cells, thereby creating a microenvironment which is favourable for the tumor to grow. In particular, PGE2 signalling via EP2 and EP4 can for example (i) suppress the cytotoxicity and cytokine production of natural killer cells, (ii) skew the polarization of tumor-associated macrophages towards tumor-promoting M2 macrophages (see for example Nakanishi Y et al Carcinogenesis 2011, 32:1333-39), (iii) regulate the activation, expansion and effector function of both Tregs (regulatory T cells) and MDSC (myeloid derived suppressor cells), which are potent immunosuppressive cells that accumulate in tumors both in patients and in experimental animal models (see for example Sharma S et al, Cancer Res 2005, 5(12):5211-20; Sinha P et al Cancer Res 2007, 67(9), 4507-4513; Obermajer N et al, Blood 2011, 118(20):5498-5505); (iv) down-regulate IFN-γ, TNF-α IL-12 and IL-2 expression in immune cells such as natural killer cells, T-cells, dendritic cells and macrophages, impairing the ability of these immune cells to induce tumor cell apoptosis and restrain tumorigenesis (see for example Bao YS et al, Int Immunopharmacol. 2011;11(10):1599-605; Kim JG and Hahn YS, Immunol Invest. 2000;29(3):257-69; Demeuere CE et al, Eur J Immunol. 1997;27(12):3526-31; Mitsuhashi M et al, J Leukoc Biol. 2004;76(2):322-32; Pockaj BA et al , Ann Surg Oncol. 2004;11(3):328-39; (v) suppress activation, IL-2 responsivness, expansion and cytotoxicity of T-cells thereby contributing to local immunsuppresion (see for example Specht C et al, Int J Cancer 200191:705-712); (vi) inhibit maturation of dendritic cells, their ability to present antigens and to produce IL-12, resulting in abortive activation of cytotoxic T-cells (see for example Ahmadi M et al, Cancer Res 2008, 68(18):7250-9; Stolina M et al, J Immunol 2000, 164:361-70); (vii) regulate tumor angiogenesis (formation of new blood vessels for nutrient and oxygen supply) by enhancing endothelial cell motility and survival as well as by increasing the expression of VEGF (vascular endothelial growth factor) (see for example Zhang Y and Daaka Y, Blood 2011;118(19):5355-64; Jain S et al, Cancer Res. 2008; 68(19):7750-9; Wang and Klein, Molecular Carcinogenesis 2007, 46:912-923; (viii) enhance tumor cell survival (via PI3K/AKT and MAPK signalling). For review see for example Kalinski P, J Immunol 2012, 188(1), 21-28; Obermajer N et al, Oncoimmunology 1(5), 762-4; Greenhough A et al, carcinogenesis 2009, 30(3), 377-86; Wang D and Dubois RN, Gut 2006, 55, 115-122; Harris SG e al Trends Immunol 2002, 22, 144-150).

Coxibs have been shown to render tumor cells more sensitive to radiation and chemotherapy and several clinical trials have been performed or are ongoing combining Coxibs with radio- and/or chemotherapy (for review see e.g Ghosh N et al, Pharmacol Rep. 2010 Mar-Apr;62(2):233-44; Davis TW et al, Am J Clin Oncol. 2003, 26(4):S58-61; see also Higgins JP et al, Cancer Biol Ther 2009, 8:1440-49).

Furthermore, there is some evidence of additive effects and/or synergy between Coxibs and epidermal growth factor receptor (EGFR) inhibitors (see for example Zhang X et al, Clin Cancer Res. 2005, 11(17):6261-9; Yamaguchi NH et al, J Gastrointest Oncol. 2014, 5(1):57-66); and with aromatase inhibitors (see for example Generali D et al, Br J Cancer. 2014;111(1):46-54; Lustberg MB et all, Clin Breast Cancer. 2011 Aug;11(4):221-7; Falandry C et al, Breast Cancer Res Treat. 2009 Aug;116(3):501-8); Chow LW et al, J Steroid Biochem Mol Biol. 2008, 111(1-2):13-7).

Moreover, additive/synergistic effects have been seen in different mouse tumor models when Aspirin (a COX1/2 inhibitor) was combined with and anti-VEGF antibody (Motz GT et al; Nat Med 2014 20(6):607) and this combination is currently under investigation in clinical trials (NCT02659384).

Recently, it has been shown that, if combined, different immunotherapeutic approaches can have enhanced anti-tumor efficacy. Due to the immune-modulatory properties of PGE2, Coxibs have thus also been used in combination with different immunotherapeutic approaches. In particular, additive or even synergistic effects could be observed when Coxibs were combined with dendritic cell vaccination in a rat glioma model and in a mouse mesothelioma or melanoma model (Zhang H et al, Oncol Res. 2013;20(10):447-55; Veltman JD et al, BMC Cancer. 2010;10:464; Toomey D et all, Vaccine. 2008 Jun 25;26(27-28):3540-9); with granulocyte-macrophage colony-stimulating factor (GM-CSF) in mouse brain tumors (Eberstål S et al, Int J Cancer. 2014 Jun 1;134(11):2748-53); with interferon gamma (IFN-γ) in brain tumors (Eberstål S et al, Cancer Immunol Immunother. 2012, 61(8):1191-9); with dendritic cell vaccination or with GM-CSF in a mouse breast cancer model (Hahn T et al, Int J Cancer. 2006,118(9):2220-31); and with adenoviral interferon beta (IFN-β) therapy in a mouse mesothelioma model (DeLong P et al, Cancer Res. 2003 Nov 15;63(22):7845-52). Along these lines, additive or even synergistic effects of Coxibs and/or EP2 and/or EP4 antagonists can also be envisaged with agents acting on cytotoxic T-lymphocyte-associated protein 4 (CTLA-4) such as anti-CTLA-4 antibodies; anti-TIM-3 antibodies, anti-Lag-3 antibodies; anti-TIGIT antibodies; or, in particular, with agents acting on programmed cell death protein 1 (PD1), such as anti-PD1 or anti-PDL1 (programmed cell death ligand 1) antibodies (Yongkui Li et al Oncoimmunology 2016, 5(2):e1074374; Zelenay S et al, Cell 2015, 162; 1-14; WO2013/090552, which indicates a synergistic effect of dual EP2 and EP4 blockade in combination with agents acting on PD1).

Adenosine is another endogenous factor with anti-inflammatory properties that is generated through the activity of ectonucleotidases, CD39 and CD73, expressed on various cell types, including regulatory T cells (Treg) (Mandapathil M et al, J Biol Chem. 2010; 285(10):7176-86). Immune cells also respond to Adenosine, because they bear receptors for ADO, which are mainly of the A2a/A2b type (Hoskin DW, et al, Int J Oncol 2008, 32:527-535). Signaling via Adenosine receptors and EP2/EP4 receptors converges on the cytoplasmic adenylyl cyclase, leading to up-regulation of cAMP. It was shown that Adenosine and PGE2 cooperate in the suppression of immune responses mediated by regulatory T cells (Mandapathil M et al, J Biol Chem. 2010; 285(36):27571-80; Caiazzo E et al, Biochem Pharmacol. 2016; 112:72-81).

Thus, the present EP2 and/or EP4 antagonists may be useful, alone, or in combination with with one or more therapeutic agents and/or chemotherapy and/or radiotherapy and/or immunotherapy; in particular in combination with chemotherapy, radiotherapy, EGFR inhibitors, aromatase inhibitors, anti-angiogenic drugs, adenosine inhibitors, immunotherapy such as especially PD1 and/or PDL1 blockade, or other targeted therapies; for the prevention / prophylaxis or treatment of cancers, notably for the prevention / prophylaxis or treatment of skin cancer including melanoma including metastatic melanoma; lung cancer including non-small cell lung cancer; bladder cancer including urinary bladder cancer, urothelial cell carcinoma; renal carcinomas including renal cell carcinoma, metastatic renal cell carcinoma, metastatic renal clear cell carcinoma; gastro-intestinal cancers including colorectal cancer, metastatic colorectal cancer, familial adenomatous polyposis (FAP), oesophageal cancer, gastric cancer, gallbladder cancer, cholangiocarcinoma, hepatocellular carcinoma, and pancreatic cancer such as pancreatic adenocarcinoma or pancreatic ductal carcinoma; endometrial cancer; ovarian cancer; cervical cancer; neuroblastoma; prostate cancer including castrate-resistant prostate cancer; brain tumors including brain metastases, malignant gliomas, glioblastoma multiforme, medulloblastoma, meningiomas; breast cancer including triple negative breast carcinoma; oral tumors; nasopharyngeal tumors; thoracic cancer; head and neck cancer; leukemias including acute myeloid leukemia, adult T-cell leukemia; carcinomas; adenocarcinomas; thyroid carcinoma including papillary thyroid carcinoma; choriocarcinoma; Ewing's sarcoma; osteosarcoma; rhabdomyosarcoma; Kaposi's sarcoma; lymphoma including Burkitt's lymphoma, Hodgkin's lymphoma, MALT lymphoma; multiple myelomas; and virally induced tumors.

In addition, selective or dual EP2 and/or EP4 antagonists may be useful in several other diseases or disorders responding for example to treatment with COX2 inhibitors, with the advantage that EP2 and/or EP4 antagonists should not possess the potential cardiovascular side effects seen with COX2 inhibitors, which are mainly due to interference with PGI2 and TXA2 synthesis (see for example Boyd MJ et al, bioorganic and medicinal chemistry letters 21, 484, 2011). For example, blockade of prostaglandin production by COX inhibitors is the treatment of choice for pain, including especially inflammatory pain and painful menstruation. Thus EP2 and/or EP4 and/or dual EP2/EP4 antagonists may be useful for the treatment of pain, especially inflammatory pain. Evidence from EP2 knockout mice suggest that EP2 antagonists can be used for the treatment of inflammatory hyperalgesia (Reinold H et al, J Clin Invest 2005, 115(3):673-9). In addition, EP4 antagonists have beneficial effect in vivo in inflammatory pain models (eg Murase A, Eur J Pharmacol 2008; Clark P, J Pharmacol Exp Ther. 2008; Maubach KA Br J Pharmacol. 2009; Colucci J Bioorg Med Chem Lett. 2010, Boyd MJ et al, Bioorg Med Chem Lett 2011, Chn Q et al Br J Phramacol 2010, Nakao K et al, J Pharmacol Exp Ther. 2007 Aug;322(2):686-94). Administration of an EP2 in combination with an EP4 antagonist showed significant, but partial inhibition of joint inflammation in mouse collagen-induced arthritis model (Honda T et al J Exp Med 2006, 203(2):325-35).

EP2 and/or dual EP2/EP4 antagonists may be of use to decrease female fertility, i.e. they have been shown to prevent pregnancy if used as contraceptive in macaques (Peluffo MC et al Hum Reprod 2014). EP2 knockout mice have decreased fertility, smaller litter sizes and reduced cumulus expansion (Matsumoto et al, Biology of reproduction 2001, 64; 1557-65; Hitzaki et al, PNAS 1999, 96(18), 10501-10506; Tilley SL J Clin Inves 1999, 103(11):1539-45; Kennedy CR et al, Nat Med 1999 5(2):217-20).

There is also rationale that EP2 and/ or EP4 antagonists may be of use to prevent or treat endometriosis: for example EP2, EP3 and EP4 and COX2 are overexpressed in endometriosis cell lines and tissues (e.g. Santulli P et al J Clin Endocrinol Metab 2014, 99(3):881-90); antagonist treatment was shown to inhibit the adhesion of endometrial cells in vitro (Lee J et al Biol Reprod 2013, 88(3):77; Lee J et al Fertil Steril 201, 93(8):2498-506); COX2 inhibitors have been shown to reduce endometric lesions in mice via EP2 (Chuang PC et al, Am J Pathol 2010, 176(2):850-60); and antagonist treatment has been shown to induce apoptosis of endometric cells in vitro (Banu SK et al, MOI endocrinol 2009, 23(8) 1291-305).

Dual EP2/EP4 antagonists, or the combination of a selective EP2 antagonists with a selective EP4 antagonist, may be of potential use for autoimmune disorders; e.g. they have been shown to be effective in mouse model for multiple sclerosis (MS) (Esaki Yet al PNAS 2010, 107(27):12233-8; Schiffmann S et al, Biochem Pharmacol. 2014, 87(4): 625-35; see also Kofler DM et al J Clin Invest 2014, 124(6):2513-22). Activation of EP2 / EP 4 signalling in cells in vitro (Kojima F et al Prostaglandins Other Lipid Mediat 2009, 89:26-33) linked dual or selective EP2 and/or EP4 antagonists to the treatment of rheumatoid arthritis. Also, elevated levels of PGE(2) have been reported in synovial fluid and cartilage from patients with osteoarthritis (OA) and it has been shown that PGE2 stimulates matrix degradation in osteoarthitis chondrocytes via the EP4 receptor (Attur M et al, J Immunol. 2008;181(7):5082-8).

EP4 overexpression is associated with enhanced inflammatory reaction in atherosclerotic plaques of patients (Cipollone F et al, Artherioscler Thromb Vasc Biol 2005, 25(9); 1925-31), thus the use of EP4 and/or dual EP2/EP4 antagonists may be indicated for plaque stabilization and prevention / prophylaxis of acute ischemic syndromes. In addition, EP4 deficiency suppresses early atherosclerosis, by compromising macrophage survival (Babaev VR et al, Cell Metab. 2008 Dec;8(6):492-501)

EP2 and/or dual EP2/EP4 antagonists may also be useful in the treatment of pneumonia: intrapulmonary administration of apoptotic cells demonstrated that PGE(2) via EP2 accounts for subsequent impairment of lung recruitment of leukocytes and clearance of Streptococcus pneumoniae, as well as enhanced generation of IL-10 in vivo (Medeiros Al et al J Exp Med 2009 206(1):61-8).

EP2 and/or dual EP2/EP4 antagonists may in addition be useful for the treatment of neurodegenerative diseases (for review see Cimino PJ et al, Curr Med Chem. 2008;15(19):1863-9). EP2 receptor accelerates progression of inflammation in a mouse model of amyotrophic lateral sclerosis (ALS) (Liang X et al, Ann Neurol 2008, 64(3):304-14); COX2 inhibitors have been shown to be neuroprotective in rodent models of stroke, Parkinson disease and ALS (for review see Liang X et al J Mol Neurosci 2007, 33(1):94-9), decreased neurotoxicity was observed in EP2 knockout mice treated with parkinsonian toxican (Jin J et al, J Neuroinflammation 2007, 4:2), PGE2 via EP2 aggravates neurodegeneration in cultured rat cells (Takadera T et al, Life Sci 2006, 78(16): 1878-83); Reduced amyloid burden was observed in Alzheimer's disease mouse model if crossed with EP2 knockout mice (Liang X et al J Neurosci 2005, 25(44):10180-7; Keene CD etal, Am J Pathol. 2010, 177(1):346-54). EP2 null mice are protected from CD14-dependent/ innate immunity mediated neuronal damage in neurodegenerative disease (Shie FS et al Glia 2005, 52(1):70-7); PGE2 via EP2 increases amyloid precursor protein (APP) expression in cultured rat microglial cells (Pooler AM et al Neurosci. Lett. 2004, 362(2):127-30). EP2 antagonist limits oxidative damage from activation of innate immunity (intracranial injection of LPS) in the brain and could be used for Alzheimer or HIV associated dementia (Montine TJ et al, J Neurochem 2002, 83(2):463-70). In an Alzheimer's disease mouse model cognitive function could be improved by genetic and pharmacological inhibition of EP4 (Hoshino T et al, J Neurochem 2012, 120(5):795-805).

EP2 and/or dual EP2/EP4 antagonists may also be useful to treat autosomal dominant polycystic kidney disease (ADPKD): PGE2 via EP2 induces cystogenesis of human renal epithelial cells; and EP2 was found to be overexpressed in patient samples (Elberg G et al, Am J Physiol Renal Physiol 2007, 293(5):F1622-32).

EP4 and/or dual EP2/EP4 antagonists may also be useful to treat osteoporosis: PGE2 stimulates bone resorption mainly via EP4 and partially via EP2 (Suzawa T et all, Endocrinology. 2000 Apr;141(4):1554-9), EP4 knockout mice show impaired bone resorption (Miyaura C et al, J Biol Chem 2000, 275(26): 19819-23) and an EP4 antagonists showed partial inhibition of PGE(2)-stimulated osteoclastogenesis and osteoclastic bone resorption (Tomita M et al, Bone. 2002 Jan;30(1):159-63).

WO2008/152093 discloses selective EP2 receptor modulators which comprise an indole ring linked to the rest of the molecule in position 3, and a pyrimidine moiety which however is not substituted with a directly linked aromatic substituent. WO2006/044732 discloses pyrimidine compounds which are modulators of PGD2 claimed to be useful e.g. in the treatment of allergic diseases. WO2008/006583 discloses pyrimidin derivatives which are ALK-5 inhibitors. WO2006/044732 and WO2008/039882 disclose certain pyrimidine derivatives as prostaglandin D2 receptor antagonists. Pyrimidin-2-yl derivatives are disclosed in WO2013/020945, WO2012/127032, WO2011/144742, Bioorg. Med. Chem 2011, 21(13) 4108-4114 and Bioorg. Med. Chem 2011, 21(1) 66-75. Certain indole-1-acetamide compounds are known as library compounds, e.g. CAS 1448123-30-5 and CAS 1448075-88-4. Further compounds which are claimed to be active as anti-cancer agents are disclosed in WO2006/128129, WO2008/008059 and Bioorg. Med. Chem 2013, 21(2), 540-546. WO2013/163190 and WO2015/058031 disclose certain DNA-PK inhibitors interacting with DNA repair processes. The disclosed compounds are thought to be useful to sensitize cancer cells, and to enhance the efficacy of both cancer chemotherapy and radiotherapy. EP1661896 discloses pyrrolopyrimidine-thione derivatives carrying a 2-aminoethyl linked substituent at the pyrrolo nitrogen, showing GSK-3 inhibitory activity instead of being modulators of the prostaglandin 2 receptor EP2.

The present invention provides novel N-substituted indole derivatives of formula (I) which are modulators of the prostaglandin 2 receptor EP2. The present compounds may, thus, as single agents or especially in combination with one or more therapeutic agents such as especially a modulator of the PGE2 receptor EP4, be useful for the prevention / prophylaxis or treatment of diseases which respond to the blockage of the EP2 receptors (or, if used in combination with a modulator of the PGE2 receptor EP4, to the blockage of both the EP2 and EP4 receptors) such as especially cancers; as well as pain including especially inflammatory pain and painful menstruation; endometriosis; acute ischemic syndromes in atherosclerotic patients; pneumonia; neurodegenerative diseases including amyotrophic lateral sclerosis, stroke; Parkinson disease, Alzheimer's disease and HIV associated dementia; autosomal dominant polycystic kidney disease; and to control female fertility.
1) A first aspect of the invention relates to compounds of the formula (I) 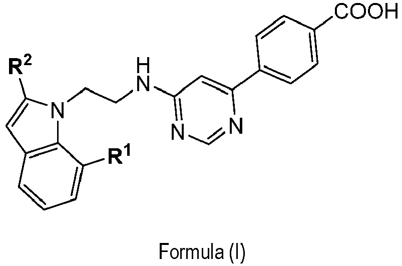 wherein
   **R¹** represents hydrogen or methyl;
   **R²** represents methyl, bromo, chloro, or cyano.

The present invention also includes isotopically labelled, especially ²H (deuterium) labelled compounds of formula (I) according to embodiments 1) to 7), which compounds are identical to the compounds of formula (I) except that one or more atoms have each been replaced by an atom having the same atomic number but an atomic mass different from the atomic mass usually found in nature. Isotopically labelled, especially ²H (deuterium) labelled compounds of formula (I) and salts thereof are within the scope of the present invention. Substitution of hydrogen with the heavier isotope ²H (deuterium) may lead to greater metabolic stability, resulting e.g. in increased *in-vivo* half-life or reduced dosage requirements, or may lead to reduced inhibition of cytochrome P450 enzymes, resulting e.g. in an improved safety profile. In one embodiment of the invention, the compounds of formula (I) are not isotopically labelled, or they are labelled only with one or more deuterium atoms. In a sub-embodiment, the compounds of formula (I) are not isotopically labelled at all. Isotopically labelled compounds of formula (I) may be prepared in analogy to the methods described hereinafter, but using the appropriate isotopic variation of suitable reagents or starting materials.

In this patent application, a bond drawn as a dotted line shows the point of attachment of the radical drawn. For example, the radical drawn below 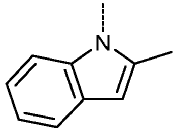 is the 2-methyl-1*H*-indol-1-yl group.

Where the plural form is used for compounds, salts, pharmaceutical compositions, diseases and the like, this is intended to mean also a single compound, salt, or the like.

Any reference to compounds of formula (I) according to embodiments 1) to 7) is to be understood as referring also to the salts (and especially the pharmaceutically acceptable salts) of such compounds, as appropriate and expedient.

The term "pharmaceutically acceptable salts" refers to salts that retain the desired biological activity of the subject compound and exhibit minimal undesired toxicological effects. Such salts include inorganic or organic acid and/or base addition salts depending on the presence of basic and/or acidic groups in the subject compound. For reference see for example "Handbook of Phramaceutical Salts. Properties, Selection and Use.", P. Heinrich Stahl, Camille G. Wermuth (Eds.), Wiley-VCH, 2008; and "Pharmaceutical Salts and Co-crystals", Johan Wouters and Luc Quéré (Eds.), RSC Publishing, 2012.

Definitions provided herein are intended to apply uniformly to the compounds of formula (I), as defined in any one of embodiments 1) to 6), and, *mutatis mutandis,* throughout the description and the claims unless an otherwise expressly set out definition provides a broader or narrower definition. It is well understood that a definition or preferred definition of a term defines and may replace the respective term independently of (and in combination with) any definition or preferred definition of any or all other terms as defined herein.

Whenever a substituent is denoted as optional, it is understood that such substituent may be absent, in which case all positions having a free valency (to which such optional substituent could have been attached to; such as for example in an aromatic ring the ring carbon atoms and / or the ring nitrogen atoms having a free valency) are substituted with hydrogen where appropriate. Likewise, in case the term "optionally" is used in the context of (ring) heteroatom(s), the term means that either the respective optional heteroatom(s), or the like, are absent (i.e. a certain moiety does not contain heteroatom(s) / is a carbocycle / or the like), or the respective optional heteroatom(s), or the like, are present as explicitly defined.

The term "halogen" means fluorine, chlorine, bromine, or iodine; especially fluorine, chlorine, or bromine; preferably fluorine or chlorine.

The term "alkyl", used alone or in combination, refers to a saturated straight or branched chain hydrocarbon group containing one to six carbon atoms. The term "(C_{x-y})alkyl" (x and y each being an integer), refers to an alkyl group as defined before, containing x to y carbon atoms. For example a (C₁₋₆)alkyl group contains from one to six carbon atoms. Examples of alkyl groups are methyl, ethyl, propyl, isopropyl, butyl, isobutyl, tert.-butyl, 3-methylbutyl, 2,2-dimethyl-propyl and 3,3-dimethyl-butyl. For avoidance of any doubt, in case a group is referred to as e.g. propyl or butyl, it is meant to be n-propyl, respectively n-butyl. Preferred are methyl and ethyl. Most preferred is methyl.

The term "alkoxy", used alone or in combination, refers to an alkyl-O- group wherein the alkyl group is as defined before. The term "(C_{x-y})alkoxy" (x and y each being an integer) refers to an alkoxy group as defined before containing x to y carbon atoms. For example a (C₁₋₄)alkoxy group means a group of the formula (C₁₋₄)alkyl-O- in which the term "(C₁₋₄)alkyl" has the previously given significance. Examples of alkoxy groups are methoxy, ethoxy, n-propoxy, isopropoxy, n-butoxy, isobutoxy, sec.-butoxy and tert.-butoxy. Preferred are ethoxy and especially methoxy.

The term "fluoroalkyl", used alone or in combination, refers to an alkyl group as defined before containing one to three carbon atoms in which one or more (and possibly all) hydrogen atoms have been replaced with fluorine. The term "(C_{x-y})fluoroalkyl" (x and y each being an integer) refers to a fluoroalkyl group as defined before containing x to y carbon atoms. For example a (C₁₋₃)fluoroalkyl group contains from one to three carbon atoms in which one to seven hydrogen atoms have been replaced with fluorine. Representative examples of fluoroalkyl groups include trifluoromethyl, 2-fluoroethyl, 2,2-difluoroethyl and 2,2,2-trifluoroethyl. Preferred are (C₁)fluoroalkyl groups such as trifluoromethyl.

The term "fluoroalkoxy", used alone or in combination, refers to an alkoxy group as defined before containing one to three carbon atoms in which one or more (and possibly all) hydrogen atoms have been replaced with fluorine. The term "(C_{x-y})fluoroalkoxy" (x and y each being an integer) refers to a fluoroalkoxy group as defined before containing x to y carbon atoms. For example a (C₁₋₃)fluoroalkoxy group contains from one to three carbon atoms in which one to seven hydrogen atoms have been replaced with fluorine. Representative examples of fluoroalkoxy groups include trifluoromethoxy, difluoromethoxy, 2-fluoroethoxy, 2,2-difluoroethoxy and 2,2,2-trifluoroethoxy. Preferred are (C₁)fluoroalkoxy groups such as trifluoromethoxy and difluoromethoxy, as well as 2,2,2-trifluoroethoxy.

The term "cycloalkyl", used alone or in combination, refers to a saturated monocyclic hydrocarbon ring containing three to six carbon atoms. The term "(C_{x-y})cycloalkyl" (x and y each being an integer), refers to a cycloalkyl group as defined before containing x to y carbon atoms. For example a (C₃₋₆)cycloalkyl group contains from three to six carbon atoms. Examples of cycloalkyl groups are cyclopropyl, cyclobutyl, cyclopentyl, cyclohexyl, and cycloheptyl. Preferred are cyclopropyl, cyclobutyl, and cyclopentyl; especially cyclopropyl.

The term "cyano" refers to a group -CN.

The compounds of Formula (I) are substituted with a carboxylic acid group -COOH; it is understood that such carboxylic acid group may be present in form of a prodrug group. Such prodrugs are encompassed in the scope of the present invention. In certain instances, compounds comprising such carboxylic acid prodrug groups may as such exhibit biological activity on the EP2 receptor, whereas in other instances, such compounds comprising such carboxylic acid prodrug groups require (e.g. enzymatic) cleavage of the prodrug to exhibit biological activity on the EP2 receptor. Prodrugs of the carboxylic acid functional group are well known in the art (see for example J. Rautio (Ed.) Prodrugs and Targeted Delivery: Towards Better ADME Properties, Volume 47, Wiley 2010,ISBN: 978-3-527-32603-7; H. Maag in Stella, V., Borchardt, R., Hageman, M., Oliyai, R., Maag, H., Tilley, J. (Eds.) Prodrugs: Challenges and Rewards, Springer 2007, ISBN 978-0-387-49785-3).

Particular examples of prodrugs, for example suitable for such -COOH groups are:
- ester groups -CO-O-**P¹** wherein **P¹** is for example (C₁₋₄)alkyl; (C₃₋₆)cycloalkyl wherein the (C₃₋₆)cycloalkyl optionally contains a ring oxygen atom; (C₃₋₆)cycloalkyl-(C₁₋₃)alkyl wherein the (C₃₋₆)cycloalkyl optionally contains a ring oxygen atom; (C₁₋₃)fluoroalkyl; hydroxy-(C₂₋₄)alkyl; or (C₁₋₄)alkoxy-(C₂₋₄)alkyl (especially **P¹** is (C₁₋₄)alkyl, in particular methyl or ethyl);
- groups -CO-NH-SO₂-**R^{S3}** wherein **R^{S3}** represents (C₁₋₄)alkyl, (C₃₋₆)cycloalkyl wherein the (C₃₋₆)cycloalkyl optionally contains a ring oxygen atom; (C₃₋₆)cycloalkyl-(C₁₋₃)alkyl wherein the (C₃₋₆)cycloalkyl optionally contains a ring oxygen atom; (C₁₋₃)fluoroalkyl, phenyl, -NH₂; (especially **R^{S3}** is (C₁₋₄)alkyl, (C₃₋₆)cycloalkyl, or phenyl; in particular methyl);
- groups - -CO-**R^{O1}** wherein **R^{O1}** represents -O-CH₂-CO-**R^{O4},** wherein **R^{O4}** repesents hydroxy, or (C₁-₄)alkoxy, or-N[(C₁₋₄)alkyl]₂ (especially -CO-O-CH₂-COOH, -CO-O-CH₂-CO-N(CH₃)₂);
- groups -CO-**R^{O1}** wherein **R^{O1}** represents -O-CH₂-O-CO-**R^{O5}**, wherein **R^{O5}** repesents (C₁₋₄)alkyl or (C₁₋₄)alkoxy (especially -CO-O-CH₂-O-CO-O-ethyl, -CO-O-CH₂-O-CO-propyl);
- groups -CO-**R^{O1}** wherein **R^{O1}** represents -O-CH₂-CH₂-N[(C₁₋₄)alkyl]₂ (especially -CO-O-CH₂-CH₂-N(CH₃)₂); and
- groups -CO-**R^{O1}** wherein **R^{O1}** represents 5-methyl-2-oxo-[1,3]dioxol-4-yl)-methyloxy-.

Whenever the word "between" is used to describe a numerical range, it is to be understood that the end points of the indicated range are explicitly included in the range. For example: if a temperature range is described to be between 40 °C and 80 °C, this means that the end points 40 °C and 80 °C are included in the range; or if a variable is defined as being an integer between 1 and 4, this means that the variable is the integer 1, 2, 3, or 4.

Unless used regarding temperatures, the term "about" placed before a numerical value "X" refers in the current application to an interval extending from X minus 10% of X to X plus 10% of X, and preferably to an interval extending from X minus 5% of X to X plus 5% of X. In the particular case of temperatures, the term "about" placed before a temperature "Y" refers in the current application to an interval extending from the temperature Y minus 10°C to Y plus 10°C, and preferably to an interval extending from Y minus 5°C to Y plus 5°C. Besides, the term "room temperature" as used herein refers to a temperature of about 25°C.

Further embodiments of the invention are presented hereinafter:
2) A second embodiment relates to compounds according to embodiment 1), wherein **R¹** represents hydrogen.
3) Another embodiment relates to compounds according to embodiment 1), wherein **R¹** represents methyl.
4) Another embodiment relates to compounds according to any one of embodiments 1) to 3), wherein **R²** represents methyl.
5) Another embodiment relates to compounds according to any one of embodiments 1) to 3), wherein **R²** represents chloro or bromo (especially chloro).
6) Another embodiment relates to compounds according to any one of embodiments 1) to 3), wherein **R²** represents cyano.
7) Another embodiment relates to most preferred compounds according to embodiment 1) which are selected from the following compounds:
   4-{6-[2-(2-Methyl-indol-1-yl)-ethylamino]-pyrimidin-4-yl}-benzoic acid;
   4-{6-[2-(2-Cyano-indol-1-yl)-ethylamino]-pyrimidin-4-yl}-benzoic acid;
   4-{6-[2-(2,7-Dimethyl-indol-1-yl)-ethylamino]-pyrimidin-4-yl}-benzoic acid;
   4-{6-[2-(2-Chloro-indol-1-yl)-ethylamino]-pyrimidin-4-yl}-benzoic acid; and
   4-{6-[2-(2-Bromo-indol-1-yl)-ethylamino]-pyrimidin-4-yl}-benzoic acid.

The compounds of formula (I) according to embodiments 1) to 7) and their pharmaceutically acceptable salts can be used as medicaments, e.g. in the form of pharmaceutical compositions for enteral (such especially oral e.g. in form of a tablet or a capsule) or parenteral administration (including topical application or inhalation).

The production of the pharmaceutical compositions can be effected in a manner which will be familiar to any person skilled in the art (see for example Remington, The Science and Practice of Pharmacy, 21st Edition (2005), Part 5, "Pharmaceutical Manufacturing" [published by Lippincott Williams & Wilkins]) by bringing the described compounds of formula (I) or their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

The present invention also relates to a method for the prevention / prophylaxis or treatment of a disease or disorder mentioned herein comprising administering to a subject a pharmaceutically active amount of a compound of formula (I) according to embodiments 1) to 7).

In a preferred embodiment of the invention, the administered amount is comprised between 1 mg and 2000 mg per day, particularly between 5 mg and 1000 mg per day, more particularly between 25 mg and 500 mg per day, especially between 50 mg and 200 mg per day.

Whenever the word "between" is used to describe a numerical range, it is to be understood that the end points of the indicated range are explicitly included in the range. For example: if a temperature range is described to be between 40 °C and 80 °C, this means that the end points 40 °C and 80 °C are included in the range; or if a variable is defined as being an integer between 1 and 4, this means that the variable is the integer 1, 2, 3, or 4.

Unless used regarding temperatures, the term "about" placed before a numerical value "X" refers in the current application to an interval extending from X minus 10% of X to X plus 10% of X, and preferably to an interval extending from X minus 5% of X to X plus 5% of X. In the particular case of temperatures, the term "about" placed before a temperature "Y" refers in the current application to an interval extending from the temperature Y minus 10 °C to Y plus 10 °C, and preferably to an interval extending from Y minus 5 °C to Y plus 5 °C.

For avoidance of any doubt, if compounds are described as useful for the prevention / prophylaxis or treatment of certain diseases, such compounds are likewise suitable for use in the preparation of a medicament for the prevention / prophylaxis or treatment of said diseases. Likewise, such compounds are also suitable in a method for the prevention / prophylaxis or treatment of such diseases, comprising administering to a subject (mammal, especially human) in need thereof, an effective amount of such compound.

The compounds of formula (I) according to embodiments 1) to 7) are useful for the prevention / prophylaxis or treatment of disorders relating to the EP2 and/or, if used in combination with a modulator of the PGE2 receptor EP4, to both the EP2 and EP4 receptors.

Compounds inhibiting the EP4 receptor are in particular the compounds 4-[[4-(5-methoxy-2-pyridinyl)phenoxy]methyl]-5-methyl-N-[(2-methylphenyl)sulfonyl]-2-furancarboxamide (BGC-20-1531, BGC20-1531; WO2004/067524); N-[[2,4-(2-ethyl-4,6-dimethyl-1H-imidazo[4,5-c]pyridin-1-yl)phenylethylamino]carbonyl]-4-methyl-benzenesulfonamide (Grapiprant, AAT-007, CJ-023423, MR-10A7, RQ-00000007, RQ-07, RQ-7-; WO2002/032900); 4-[(1S)-1-[[[3-(difluoromethyl)-1-methyl-5-[3-(trifluoromethyl)phenoxy]-1H-pyrazol-4-yl]carbonyl]amino]ethyl]-benzoic acid (E-7046, ER-886046-00; WO2012/039972); CR-6086 (WO2012/076063); ONO-4578 (WO2016/111347); and 4-[1(S)-[5-Chloro-2-(3-fluorophenoxy)pyridin-3-ylcarboxamido]ethyl]benzoic acid (AAT-008, RQ-08, RQ-00000008; WO2005/021508); as well as compounds disclosed in WO2017/066633; WO2017/014323; WO2016/111347; WO2016/021742; WO2015/179615; WO2015/147020; WO2015/091475; WO2015/094912; WO2015/094902; WO2014/200075; WO2014/186218; WO2014/126746; WO2014/122267; WO2014/086739; WO2014/004230; WO2014/004229; WO2013/004290; WO2012/103071; WO2012/076063; WO2012/043634; WO2012/039972; WO2010/034110; WO2010/032123; WO2010/019796; WO2009/139373; WO2009/005076; WO2008/123207; WO2008/116304; WO2008/104055; WO2008/017164; WO2007/143825; WO2007/121578; WO2006/122403; WO2005/105733; WO2005/105732; WO2005/037812; WO2005/021508; WO2004/067524; WO2003/099857; WO2003/086390; WO2003/087061; WO2002/064564; WO2002/050032; WO2002/050033; WO2002/032422; WO2001/072302.

Certain compounds of formula (I) according to embodiments 1) to 7) exhibit their biological activity as modulators of the prostaglandin 2 receptor EP2 in a biological environment, (i.e. in the presence of one or more enzymes capable of breaking a covalent bond linked to a carbonyl group such as an amidase, an esterase or any suitable equivalent thereof capable of removing a prodrug group from a carboxylic acid group.

Diseases or disorders relating to the EP2 and/or, if such compound is used in combination with a modulator of the PGE2 receptor EP4, to both the EP2 and EP4 receptors are especially
- cancer (notably melanoma including metastatic melanoma; lung cancer including non-small cell lung cancer; bladder cancer including urinary bladder cancer, urothelial cell carcinoma; renal carcinomas including renal cell carcinoma, metastatic renal cell carcinoma, metastatic renal clear cell carcinoma; gastro-intestinal cancers including colorectal cancer, metastatic colorectal cancer, familial adenomatous polyposis (FAP), oesophageal cancer, gastric cancer, gallbladder cancer, cholangiocarcinoma, hepatocellular carcinoma, and pancreatic cancer such as pancreatic adenocarcinoma or pancreatic ductal carcinoma; endometrial cancer; ovarian cancer; cervical cancer; neuroblastoma; prostate cancer including castrate-resistant prostate cancer; brain tumors including brain metastases, malignant gliomas, glioblastoma multiforme, medulloblastoma, meningiomas; breast cancer including triple negative breast carcinoma; oral tumors; nasopharyngeal tumors; thoracic cancer; head and neck cancer; leukemias including acute myeloid leukemia, adult T-cell leukemia; carcinomas; adenocarcinomas; thyroid carcinoma including papillary thyroid carcinoma; choriocarcinoma; Ewing's sarcoma; osteosarcoma; rhabdomyosarcoma; Kaposi's sarcoma; lymphoma including Burkitt's lymphoma, Hodgkin's lymphoma, MALT lymphoma; multiple myelomas; and virally induced tumors; especially melanoma; lung cancer; bladder cancer; renal carcinomas; gastro-intestinal cancers; endometrial cancer; ovarian cancer; cervical cancer; and neuroblastoma);
as well as further diseases or disorders relating to the EP2 and/or EP4 receptors such as:
- pain (notably inflammatory pain and painful menstruation);
- endometriosis;
- autosomal dominant polycystic kidney disease;
- acute ischemic syndromes in atherosclerotic patients;
- pneumonia; and
- neurodegenerative diseases including amyotrophic lateral sclerosis, stroke; Parkinson disease, Alzheimer's disease and HIV associated dementia;
- and EP2 and/or EP4 antagonists may further be used to control female fertility.

Further diseases or disorders relating to the EP2 and/or EP4 receptors are autoimmune disorders such as especially multiple sclerosis, rheumatoid arthritis and osteoarthritis; and osteoporosis.

The compounds of formula (I) according to any one of embodiments 1) to 7) are in particular useful as therapeutic agents for the prevention / prophylaxis or treatment of a cancer. They may be used as single therapeutic agents, wherein for the prevention / prophylaxis or treatment of a cancer said compounds are used preferably in combination with a modulator of the PGE2 receptor EP4; and, in addition, optionally in combination with one or more chemotherapy agents and / or radiotherapy and / or targeted therapy. Such combined treatment may be effected simultaneously, separately, or over a period of time.

The invention, thus, also relates to pharmaceutical compositions comprising a pharmaceutically acceptable carrier material, and:
- a compound of formula (I) according to any one of embodiments 1) to 7); and/or
- a modulator of the PGE2 receptor EP4; and/or
- and one or more cytotoxic chemotherapy agents.

The invention, thus, further relates to a kit comprising
- a pharmaceutical composition, said composition comprising a pharmaceutically acceptable carrier material, and: 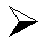 a compound of formula (I) according to any one of embodiments 1) to 7);
- and instructions how to use said pharmaceutical composition for the prevention / prophylaxis or the treatment of a cancer, in combination with chemotherapy and / or radiotherapy and / or targeted therapy.

The terms "radiotherapy" or "radiation therapy" or "radiation oncology", refer to the medical use of ionizing radiation in the prevention / prophylaxis (adjuvant therapy) and / or treatment of cancer; including external and internal radiotherapy.

The term "targeted therapy" refers to the prevention / prophylaxis (adjuvant therapy) and / or treatment of cancer with one or more anti-neoplastic agents such as small molecules or antibodies which act on specific types of cancer cells or stromal cells. Some targeted therapies block the action of certain enzymes, proteins, or other molecules involved in the growth and spread of cancer cells. Other types of targeted therapies help the immune system kill cancer cells (immunotherapies); or inhibit angiogenesis, the growth and formation of new blood vessels in the tumor; or deliver toxic substances directly to cancer cells and kill them. An example of a targeted therapy which is in particular suitable to be combined with the compounds of the present invention is immunotherapy, especially immunotherapy targeting the progammed cell death receptor 1 (PD-1 receptor) or its ligand PD-L1 (Zelenay et al., 2015, Cell 162, 1-14; Yongkui Li et al., Oncoimmunology 2016, 5(2):e1074374).

When used in combination with the compounds of formula (I), the term "targeted therapy" especially refers to agents such as:
a) Epidermal growth factor receptor (EGFR) inhibitors or blocking antibodies (for example Gefitinib, Erlotinib, Afatinib, Icotinib, Lapatinib, Panitumumab, Zalutumumab, Nimotuzumab, Matuzumab and Cetuximab);
b) RAS/RAF/MEK pathway inhibitors (for example Vemurafenib, Sorafenib, Dabrafenib,GDC-0879, PLX-4720, LGX818, RG7304, Trametinib (GSK1120212), Cobimetinib (GDC-0973/XL518), Binimetinib (MEK162, ARRY-162), Selumetinib (AZD6244));
c) Aromatase inhibitors (for example Exemestane, Letrozole, Anastrozole, Vorozole, Formestane, Fadrozole);
d) Angiogenesis inhibitors, especially VEGF signalling inhibitors such as Bevacuzimab (Avastin), Ramucirumab , Sorafenib or Axitinib;
e) Immune Checkpoint inhibitors (for example: anti-PD1 antibodies such as Pembrolizumab (Lambrolizumab, MK-3475), Nivolumab, Pidilizumab (CT-011), AMP-514/MED10680, PDR001, SHR-1210; REGN2810, BGBA317; fusion proteins targeting PD-1 such as AMP-224; small molecule anti-PD1 agents such as for example compounds disclosed in WO2015/033299, WO2015/044900 and WO2015/034820; anti-PD1L antibodies, such as BMS-936559, atezolizumab (MPDL3280A, RG7446), MEDI4736, avelumab (MSB0010718C), durvalumab (MEDI4736); anti-PDL2 antibodies, such as AMP224; anti-CTLA-4 antibodies, such as ipilimumab, tremilmumab; anti-Lymphocyte-activation gene 3 (LAG-3) antibodies, such as BMS-986016, IMP701, MK-4280, ImmuFact IMP321; anti T cell immunoglobulin mucin-3 (TIM-3) antibodies, such as MBG453; anti-CD137/4-1BB antibodies, such as BMS-663513 / urelumab, PF-05082566; anti T cell immunoreceptor with Ig and ITIM domains (TIGIT) antibodies, such as RG6058 (anti-TIGIT, MTIG7192A);
f) Vaccination approaches (for example dendritic cell vaccination, peptide or protein vaccination (for example with gp100 peptide or MAGE-A3 peptide);
g) Re-introduction of patient derived or allogenic (non-self) cancer cells genetically modified to secrete immunomodulatory factors such as granulocyte monocyte colony stimulating factor (GMCSF) gene-transfected tumor cell vaccine (GVAX) or Fms-related tyrosine kinase 3 (Flt-3) ligand gene-transfected tumor cell vaccine (FVAX),or Toll like receptor enhanced GM-CSF tumor based vaccine (TEGVAX);
h) T-cell based adoptive immunotherapies, including chimeric antigen receptor (CAR) engineered T-cells (for example CTL019);
i) Cytokine or immunocytokine based therapy (for example Interferon alpha, interferon beta, interferon gamma, interleukin 2, interleukin 15);
j) Toll-like receptor (TLR) agonists (for example resiquimod, imiquimod, glucopyranosyl lipid A, CpG oligodesoxynucleotides);
k) Thalidomide analogues (for example Lenalidomide, Pomalidomide);
l) Indoleamin-2,3-Dioxgenase (IDO) and/or Tryptophane-2,3-Dioxygenase (TDO) inhibitors (for example RG6078 / NLG919 / GDC-0919; Indoximod / 1MT (1-methyltryptophan), INCB024360 / Epacadostat, PF-06840003 (EOS200271), F001287);
m) Activators of T-cell co-stimulatory receptors (for example anti-OX40/CD134 (Tumor necrosis factor receptor superfamily, member 4, such as RG7888 (MOXR0916), 9B12; MEDI6469, GSK3174998, MEDI0562), anti OX40-Ligand/CD252; anti-glucocorticoid-induced TNFR family related gene (GITR) (such as TRX518, MEDI1873, MK-4166, BMS-986156), anti-CD40 (TNF receptor superfamily member 5) antibodies (such as Dacetuzumab (SGN-40), HCD122, CP-870,893, RG7876, ADC-1013, APX005M, SEA-CD40); anti-CD40-Ligand antibodies (such as BG9588); anti-CD27 antibodies such as Varlilumab);
n) Molecules binding a tumor specific antigen as well as a T-cell surface marker such as bispecific antibodies (for example RG7802 targeting CEA and CD3) or antibody fragments, antibody mimetic proteins such as designed ankyrin repeat proteins (DARPINS), bispecific T-cell engager (BITE, for example AMG103, AMG330);
o) Antibodies or small molecular weight inhibitors targeting colony-stimulating factor-1 receptor (CSF-1R) (for example Emactuzumab (RG7155), Cabiralizumab (FPA-008), PLX3397);
p) Agents targeting immune cell check points on natural killer cells such as antibodies against Killer-cell immunoglobulin-like receptors (KIR) for example Lirilumab (IPH2102/BMS-986015);
q) Agents targeting the Adenosine receptors or the ectonucleases CD39 and CD73 that convert ATP to Adenosine, such as MEDI9447 (anti-CD73 antibody), PBF-509; CPI-444 (Adenosine A2a receptor antagonist).

When used in combination with the compounds of formula (I), immune checkpoint inhibitors such as those listed under d), and especially those targeting the progammed cell death receptor 1 (PD-1 receptor) or its ligand PD-L1, are preferred.

The term "chemotherapy" refers to the treatment of cancer with one or more cytotoxic anti-neoplastic agents ("cytotoxic chemotherapy agents"). Chemotherapy is often used in conjunction with other cancer treatments, such as radiation therapy or surgery. The term especially refers to conventional cytotoxic chemotherapeutic agents which act by killing cells that divide rapidly, one of the main properties of most cancer cells. Chemotherapy may use one drug at a time (single-agent chemotherapy) or several drugs at once (combination chemotherapy or polychemotherapy). Chemotherapy using drugs that convert to cytotoxic activity only upon light exposure is called photochemotherapy or photodynamic therapy.

The term "cytotoxic chemotherapy agent" or "chemotherapy agent" as used herein refers to an active anti-neoplastic agent inducing apoptosis or necrotic cell death. When used in combination with the compounds of formula (I), the term especially refers to conventional cytotoxic chemotherapy agents such as:
a) alkylating agents (for example mechlorethamine, chlorambucil, cyclophosphamide, ifosfamide, streptozocin, carmustine, lomustine, melphalan, dacarbazine, temozolomide, fotemustine, thiotepa or altretamine; especially cyclophosphamide, carmustine, melphalan, dacarbazine, or temozolomide);
b) platinum drugs (especially cisplatin, carboplatin or oxaliplatin);
c) antimetabolite drugs (for example 5-fluorouracil, folic acid/leucovorin, capecitabine, 6-mercaptopurine, methotrexate, gemcitabine, cytarabine, fludarabine or pemetrexed; especially 5-fluorouracil, folic acid/leucovorin, capecitabine, methotrexate, gemcitabine or pemetrexed);
d) anti-tumor antibiotics (for example daunorubicin, doxorubicin, epirubicin, idarubicin, actinomycin-D, bleomycin, mitomycin-C or mitoxantrone; especially doxorubicin);
e) mitotic inhibitors (for example paclitaxel, docetaxel, ixabepilone, vinblastine, vincristine, vinorelbine, vindesine or estramustine; especially paclitaxel, docetaxel, ixabepilone or, vincristine); or
f) topoisomerase inhibitors (for example etoposide, teniposide, topotecan, irinotecan, diflomotecan or elomotecan; especially etoposide or irinotecan).

When used in combination with the compounds of formula (I), preferred cytotoxic chemotherapy agents are the above-mentioned alkylating agents (notably fotemustine,cyclophosphamide, ifosfamide, carmustine, dacarbazine and prodrugs thereof such as especially temozolomide or pharmaceutically acceptable salts of these compounds; in particular temozolomide); mitotic inhibitors (notably paclitaxel, docetaxel, ixabepilone,; or pharmaceutically acceptable salts of these compounds; in particular paclitaxel); platinum drugs (notably cisplatin, oxaliplatin and carboplatin); as well etoposide and gemcitabine.

Chemotherapy may be given with a curative intent or it may aim to prolong life or to palliate symptoms.
- Combined modality chemotherapy is the use of drugs with other cancer treatments, such as radiation therapy or surgery.
- Induction chemotherapy is the first line treatment of cancer with a chemotherapeutic drug. This type of chemotherapy is used for curative intent.
- Consolidation chemotherapy is the given after remission in order to prolong the overall disease free time and improve overall survival. The drug that is administered is the same as the drug that achieved remission.
- Intensification chemotherapy is identical to consolidation chemotherapy but a different drug than the induction chemotherapy is used.
- Combination chemotherapy involves treating a patient with a number of different drugs simultaneously. The drugs differ in their mechanism and side effects. The biggest advantage is minimising the chances of resistance developing to any one agent. Also, the drugs can often be used at lower doses, reducing toxicity.
- Neoadjuvant chemotherapy is given prior to a local treatment such as surgery, and is designed to shrink the primary tumor. It is also given to cancers with a high risk of micrometastatic disease.
- Adjuvant chemotherapy is given after a local treatment (radiotherapy or surgery). It can be used when there is little evidence of cancer present, but there is risk of recurrence. It is also useful in killing any cancerous cells that have spread to other parts of the body. These micrometastases can be treated with adjuvant chemotherapy and can reduce relapse rates caused by these disseminated cells.
- Maintenance chemotherapy is a repeated low-dose treatment to prolong remission.
- Salvage chemotherapy or palliative chemotherapy is given without curative intent, but simply to decrease tumor load and increase life expectancy. For these regimens, a better toxicity profile is generally expected.

"Simultaneously", when referring to an administration type, means in the present application that the administration type concerned consists in the administration of two or more active ingredients and/or treatments at approximately the same time; wherein it is understood that a simultaneous administration will lead to exposure of the subject to the two or more active ingredients and/or treatments at the same time. When administered simultaneously, said two or more active ingredients may be administered in a fixed dose combination, or in an equivalent non-fixed dose combination (e.g. by using two or more different pharmaceutical compositions to be administered by the same route of administration at approximately the same time), or by a non-fixed dose combination using two or more different routes of administration; wherein said administration leads to essentially simultaneous exposure of the subject to the two or more active ingredients and/or treatments. For example, when used in combination with chemotherapy and/or suitable targeted therapy, the present EP2/EP4 antagonists would possibly be used "simultaneously".

"Fixed dose combination", when referring to an administration type, means in the present application that the administration type concerned consists in the administration of one single pharmaceutical composition comprising the two or more active ingredients.

"Separately", when referring to an administration type, means in the present application that the administration type concerned consists in the administration of two or more active ingredients and/or treatments at different points in time; wherein it is understood that a separate administration will lead to a treatment phase (e.g. at least 1 hour, notably at least 6 hours, especially at least 12 hours) where the subject is exposed to the two or more active ingredients and/or treatments at the same time; but a separate administration may also lead to a treatment phase where for a certain period of time (e.g. at least 12 hours, especially at least one day) the subject is exposed to only one of the two or more active ingredients and/or treatments. Separate administration especially refers to situations wherein at least one of the active ingredients and/or treatments is given with a periodicity substantially different from daily (such as once or twice daily) administration (e.g. wherein one active ingredient and/or treatment is given e.g. once or twice a day, and another is given e.g. every other day, or once a week or at even longer distances). For example, when used in combination with radiotherapy, the present EP2/EP4 antagonists would possibly be used "separately".

By administration "over a period of time" is meant in the present application the subsequent administration of two or more active ingredients and/or treatments at different times. The term in particular refers to an administration method according to which the entire administration of one of the active ingredients and/or treatments is completed before the administration of the other / the others begins. In this way it is possible to administer one of the active ingredients and/or treatments for several months before administering the other active ingredient(s) and/or treatment(s).

Administration "over a period of time" also encompasses situations wherein the compound of formula (I) would be used in a treatment that starts after termination of an initial chemotherapeutic (for example an induction chemotherapy) and/or radiotherapeutic treatment and/or targeted therapy treatment, wherein optionally said treatment would be in combination with a further / an ongoing chemotherapeutic and/or radiotherapeutic treatment and/or targeted therapy treatment (for example in combination with a consolidation chemotherapy, an intensification chemotherapy, an adjuvant chemotherapy, or a maintenance chemotherapy; or radiotherapeutic equivalents thereof); wherein such further / ongoing chemotherapeutic and/or radiotherapeutic treatment and/or targeted therapy treatment would be simultaneously, separately, or over a period of time in the sense of "not given with the same periodicity".

The compounds of formula (I) as defined in embodiments 1) to 7), especially in combination with a modulator of the PGE2 receptor EP4, are also useful in a method of modulating an immune response in a subject having a tumor, comprising the administration of an effective amount of the compound of formula (I); wherein said effective amount reactivates the immune system in the tumor of said subject; wherein especially said effective amount:
- counteracts the polarization of tumor-associated macrophages towards tumor-promoting M2 macrophages; and/or
- down-regulates the activation, expansion and/or the effector function of immunosuppressive cells that have accumulated in a tumor (especially of regulatory T cells (Tregs) and/or myeloid derived suppressor cells (MDSC)); and/or
- up-regulates IFN-γ and/or TNF-α and/or IL-12 and/or IL-2 expression in immune cells such as natural killer cells, T-cells, dendritic cells and macrophages (leading to the induction of tumor cell apoptosis and/or restrained tumorigenesis); and/or
- directly or indirectly counteracts the suppressed activation, IL-2 responsiveness and expansion of cytotoxic T-cells (thereby decreasing local immunsuppression).

The compounds of formula (I) as defined in embodiments 1) to 7), especially in combination with a modulator of the PGE2 receptor EP4, are also useful in a method of diminishing tumor growth and/or reducing tumor size in a subject having a tumor, comprising the administration of an effective amount of the compound of formula (I); wherein said effective amount down-regulates tumor angiogenesis (especially by decreasing endothelial cell motility and/or survival, and/or by decreasing the expression of VEGF (vascular endothelial growth factor)); and/or wherein said effective amount diminishes tumor cell survival and/or induces tumor cell apoptosis (especially via inhibition of PI3K/AKT and MAPK signalling).

The compounds of formula (I) as defined in embodiments 1) to 7), especially in combination with a modulator of the PGE2 receptor EP4, are also useful in a method of modulating an immmune response in a subject having a tumor, comprising the administration of an effective amount of the compound of formula (I); wherein said effective amount reactivates the immune system in the tumor of said subject; wherein said effective amount activates the cytotoxicity and cytokine production of natural killer cells and/or cytotoxic T-cells.

### Experimental Part

### I. Chemistry

All temperatures are stated in °C. Commercially available starting materials were used as received without further purification. Unless otherwise specified, all reactions were carried out in oven-dried glassware under an atmosphere of nitrogen. Compounds were purified by flash column chromatography on silica gel or by preparative HPLC. Compounds described in the invention are characterised by LC-MS data (retention time t_{R} is given in min; molecular weight obtained from the mass spectrum is given in g/mol) using the conditions listed below. In cases where compounds of the present invention appear as a mixture of conformational isomers, particularly visible in their LC-MS spectra, the retention time of the most abundant conformer is given.

### Analytical LC-MS equipment:

HPLC pump: Binary gradient pump, Agilent G4220A or equivalent
Autosampler: Gilson LH215 (with Gilson 845z injector) or equivalent
Column compartment: Dionex TCC-3000RS or equivalent
Degasser: Dionex SRD-3200 or equivalent
Make-up pump: Dionex HPG-3200SD or equivalent
DAD detector: Agilent G4212A or equivalent
MS detector: Single quadrupole mass analyzer, Thermo Finnigan MSQPlus or equivalent
ELS detector: Sedere SEDEX 90 or equivalent

### LC-MS Method A

Column: Zorbax SB-aq (3.5 µm, 4.6 x 50 mm). Conditions: MeCN [eluent A]; water + 0.04% TFA [eluent B].
Gradient: 95% B → 5% B over 1.5 min (flow: 4.5 mL/min). Detection: UV/Vis + MS, t_{R} is given in min.

### Preparative HPLC equipment:

Gilson 333/334 HPLC pump equipped with Gilson LH215, Dionex SRD-3200 degasser,
Dionex ISO-3100A make-up pump, Dionex DAD-3000 DAD detector, Single quadrupole mass analyzer MS detector, Thermo Finnigan MSQ Plus, MRA100-000 flow splitter, Polymer Laboratories PL-ELS1000 ELS detector

### Preparative HPLC with basic conditions

Column: Waters XBridge (10 µm, 75 x 30 mm). Conditions: MeCN [eluent A]; water + 0.5% NH₄OH (25% aq.) [eluent B]; Gradient see **Table 1** (flow: 75 mL/min), the starting percentage of Eluent A (x) is determined depending on the polarity of the compound to purify. Detection: UV/Vis + MS

**Table 1**

| t (min) | 0 | 0.01 | 4.0 | 6.0 | 6.2 | 6.6 |
|---|---|---|---|---|---|---|
| Eluent A (%) | x | x | 95 | 95 | x | x |
| Eluent B (%) | 100-x | 100-x | 5 | 5 | 100-x | 100-x |

### Preparative HPLC with acidic conditions

Column: Waters Atlantis T3 (10 µm, 75 x 30 mm). Conditions: MeCN [eluent A]; water + 0.5% HCO₂H [eluent B]; Gradient see **Table 2** (flow: 75 mL/min), the starting percentage of Eluent A (x) is determined depending on the polarity of the compound to purify. Detection: UV/Vis + MS

**Table 2**

| t (min) | 0 | 0.01 | 4.0 | 6.0 | 6.2 | 6.6 |
|---|---|---|---|---|---|---|
| Eluent A (%) | x | x | 95 | 95 | x | x |
| Eluent B (%) | 100-x | 100-x | 5 | 5 | 100-x | 100-x |

### Abbreviations (as used hereinbefore or hereinafter):

- aq.: aqueous
- atm: atmosphere
- boc: tert-butyloxycarbonyl
- d: days
- DCM: dichloromethane
- DIPEA: diisopropyl-ethylamine, Hünig's base
- DMF: dimethylformamide
- DMSO: dimethylsulfoxide
- Et₂O: diethylether
- EtOAc: ethyl acetate
- EtOH: ethanol
- Ex.: example
- FC: flash chromatography on silica gel
- h: hour(s)
- hept: heptane(s)
- HPLC: high performance liquid chromatography
- LC-MS: liquid chromatography - mass spectrometry
- Lit.: Literature
- MeCN: acetonitrile
- MeOH: methanol
- mL: milliliter
- min: minute(s)
- MW: microwave
- Ph: phenyl
- PPh₃: triphenyl phosphine
- prep.: preparative
- RM: reaction mixture
- RT: room temperature
- s: second(s)
- sat.: saturated (if not indicated otherwise: sat. aq.)
- tBu: tert-butyl = tertiary butyl
- TEA: triethylamine
- TFA: trifluoroacetic acid
- THF: tetrahydrofuran
- TLC: thin layer chromatography
- t_{R}: retention time
- triflate: *trifluoromethanesulfonate*

### A- Preparation of pyrimidine halide derivatives of formula (III)

### A.1. 6-Chloro-N-(2-(2-methyl-1H-indol-1-yl)ethyl)pyrimidin-4-amine

To a solution of 4,6-dichloropyrimidine (3.00 g, 20.1 mmol) in 2-propanol (50 mL) at RT is added 2-(2-methyl-1H-indol-1-yl)ethan-1-amine (3.68 g, 21.1 mmol) and TEA (3.08 mL, 22.2 mmol). The resulting mixture is refluxed for 2h, then allowed to cool to RT and concentrated under reduced pressure. The residue is partitioned between sat. aq. NaHCO₃ solution and EtOAc. The layers are separated and the aqueous layer is extracted once more with EtOAc. The combined organic layers are washed with water, brine, dried over MgSO₄, filtered and the solvent is removed in vacuo yielding the desired product as a yellow powder (5.45 g, 94%). LC-MS A: t_{R} = 0.87 min; [M+H]⁺ = 287.13.

### A.1.1. 2-(2-Methyl-1H-indol-1-yl)ethan-1-amine

To a solution of 2-methylindole (10.04 g, 75 mmol) in toluene (200 mL) are added 2-chloroethylamine hydrochloride (17.4 g, 150 mmol), freshly powdered NaOH (21.00 g, 525 mmol) and tetrabutyl ammonium hydrogen sulfate (2.037 g, 6 mmol). The resulting mixture is heated up to reflux and stirred for 17h. It is then cooled down to RT, and filtered through a filter paper. The residue is triturated twice with toluene, and filtrated. The filtrate is concentrated under reduced pressure, and the residue is purified by FC, using a gradient of DCM/MeOH from 100:0 to 95:5. After concentration of the product containing fractions, the title compound (13.2 g, 99%) is obtained as a yellow resin: LC-MS A: t_{R} = 0.54 min; [M+H]+ = 175.31.

### A.2. 1-(2-((6-Chloropyrimidin-4-yl)amino)ethyl)-1H-indole-2-carbonitrile

The title compound is prepared according to the synthesis of A.1. described above using 2-(2-cyano-1H-indol-1-yl)ethan-1-aminium 2,2,2-trifluoroacetate; LC-MS A: t_{R} = 0.85 min; [M+H]⁺ = 298.05.

### A.2.1. 2-(2-Cyano-1H-indol-1-yl)ethan-1-aminium 2,2,2-trifluoroacetate

A solution of tert-butyl (2-(2-cyano-1H-indol-1-yl)ethyl)carbamate (2.08 g, 6.56 mmol) in DCM (20 mL) is treated with TFA (20 mL) and the RM is stirred for 1h at RT. The solvents are removed under vacuum. The residue is triturated three times in Et₂O, affording the title compound as a beige powder (1.56 g, 81%). LC-MS A: t_{R} = 0.82 min; [M+H]+ = 186.25.

### A.2.2. Tert-butyl (2-(2-cyano-1H-indol-1-yl)ethyl)carbamate

NaH (0.27 g, 6.75 mmol) is added portionwise to a solution of 1H-indole-2-carbonitrile (0.80 g, 5.63 mmol) in DMF (25 mL) and the RM is stirred at RT for 15 min. A solution of N-Boc-2-bromoethyl-amine (1.30 g, 5.63 mmol) in DMF (10 mL) is added dropwise, and the RM is heated up to 85°C and stirred at this temperature for 17 h, then cooled at RT and partitioned between Et₂O and H₂O. The aqueous layer is re-extracted with Et₂O (x3). The combined organic layers are dried over MgSO₄, filtered and concentrated under reduced pressure, affording the title compound as a brown oil. LC-MS A: t_{R} = 0.90 min; [M+H-Boc]⁺ = 186.27.

### A.3. 6-Chloro-N-(2-(2,7-dimethyl-1H-indol-1-yl)ethyl)pyrimidin-4-amine

The title compound is prepared according to the synthesis of A.1. described above using 2-(2-methyl-1H-indol-1-yl)ethan-1-amine; LC-MS A: t_{R} = 0.91 min; [M+H]⁺ = 301.19.

### A.3.1. 2-(2-Methyl-1H-indol-1-yl)ethan-1-amine

The title compound is prepared according to the synthesis of A.1.1. described above using 2,7-dimethylindole; LC-MS A: t_{R} = 0.58 min; [M+H]⁺ = 189.26.

### B- Preparation of examples

### General procedure A: Suzuki coupling with Pd(PPh₃)₄

A mixture of the respective pyrimidine halide derivative (II) (0.15 mmol), 4-carboxyphenylboronic acid (0.18 mmol), and K₂CO₃ 2M (0.3 mL, 0.6 mmol) in ethanol (3 mL) is purged with argon, tetrakis-(triphenylphosphine)-palladium (0.0075 mmol) is added, and the RM is heated at 90°C overnight. Alternatively, the reaction can be performed in a microwave apparatus, at 120°C for 10 - 30 min. The RM is filtered through a 0.45 um Glass MicroFiber filter, washed with EtOH/MeCN and DMF. The filtrate is purified either by preparative HPLC or FC. Alternatively, it is diluted with water, if needed the pH is adjusted, and extracted with EtOAc (3x). The combined organic extracts are dried (MgSO₄) and concentrated under reduced pressure. The residue is purified by preparative HPLC or by FC.

### Example 1: 4-{6-[2-(2-Methyl-indol-1-yl)-ethylamino]-pyrimidin-4-yl}-benzoic acid

The title compound is prepared according to the general procedure A described above using 6-chloro-N-(2-(2-methyl-1H-indol-1-yl)ethyl)pyrimidin-4-amine (A.1.) and obtained as an off-white solid; LC-MS A: t_{R} = 0.67 min; [M+H]⁺ = 373.09.

### Example 2: 4-{6-[2-(2-Cyano-indol-1-yl)-ethylamino]-pyrimidin-4-yl}-benzoic acid

The title compound is prepared according to the general procedure A described above using 1-(2-((6-chloropyrimidin-4-yl)amino)ethyl)-1H-indole-2-carbonitrile (A.2.) and obtained as a white solid; LC-MS A: t_{R} = 0.56 min; [M+H]⁺ = 384.16.

### Example 3: 4-{6-[2-(2,7-Dimethyl-indol-1-yl)-ethylamino]-pyrimidin-4-yl}-benzoic acid

The title compound is prepared according to the general procedure A described above using 6-chloro-N-(2-(2,7-dimethyl-1H-indol-1-yl)ethyl)pyrimidin-4-amine (A.3.) and obtained as a pale yellow solid; LC-MS A: t_{R} = 0.69 min; [M+H]⁺ = 386.92.

### Example 4: 4-{6-[2-(2-Chloro-indol-1-yl)-ethylamino]-pyrimidin-4-yl}-benzoic acid

A MW-vial is charged with tert-butyl 4-(6-((2-(2-oxoindolin-1-yl)ethyl)amino)pyrimidin-4-yl)benzoate (200 mg, 0.465 mmol), DCM (3 mL) and POCl3 (0.0848 mL, 0.929 mmol), it is sealed and stirred under reflux for 6h. The RM is cooled to 0°C and carefully quenched with NaOH 32% until basic pH then additional water is carefully added. The aqueous layer is extracted with DCM (x 3). Organic layers are washed with brine, dried over MgSO₄. Filtered and concentrated under reduced pressure. MeOH is added and the solvent is removed under reduced pressure. The residue is dissolved in ethanol (2 mL) and H2O (1 mL), lithium hydroxide monohydrate (101 mg, 2.38 mmol) is added and the mixture is heated at 105°C for 1h. The reaction mixture is filtered over a 0.45 um Glass MicroFiber filter and purified by basic prep HPLC to afford the crude title compound as a white solid (16 mg, 9%). LC-MS A: t_{R} = 0.69 min; [M+H]+ = 393.13.

### a) Tert-butyl 4-(6-((2-(2-oxoindolin-1-yl)ethyl)amino)pyrimidin-4-yl)benzoate

The title compound is prepared according to the general procedure A described above using 1-(2-((6-chloropyrimidin-4-yl)amino)ethyl)indolin-2-one and 4-tert-butoxycarbonylphenylboronic acid; LC-MS A: t_{R} = 0.75 min; [M+H]⁺ = 431.07.

### b) 1-(2-((6-Chloropyrimidin-4-yl)amino)ethyl)indolin-2-one

The title compound is prepared according to the synthesis of A.1. described above using 1-(2-aminoethyl)indolin-2-one; LC-MS A: t_{R} = 0.70 min; [M+H]⁺ = 289.13.

### Example 5: 4-{6-[2-(2-Bromo-indol-1-yl)-ethylamino]-pyrimidin-4-yl}-benzoic acid

A MW-vial is charged with ethyl 4-(6-((2-(2-oxoindolin-1-yl)ethyl)amino)pyrimidin-4-yl)benzoate (60 mg, 0.149 mmol), DCM (2 mL) and POBr3 (64 mg, 0.224 mmol), it is sealed and stirred under reflux for 1h. The RM is cooled to RT, imidazole (12.3 mg, 0.179 mmol) is added, and the RM is refluxed for 48h. The RM is cooled and carefully quenched with sat. aq. NaHCO3 and extracted with DCM (x 3). The organic layers are washed with brine, dried over MgSO₄, filtered and concentrated under reduced pressure. The residue is dissolved in ethanol (2 mL) and H2O (1 mL), lithium hydroxide monohydrate (35 mg, 0.83 mmol) is added and the mixture is refluxed overnight. The reaction mixture is filtered over a 0.45 um Glass MicroFiber filter and purified by basic prep HPLC to afford the crude title compound as a yellow solid (1mg, 1%). LC-MS A: t_{R} = 0.69 min; [M+H]+ = 438.85.

### a) Ethyl 4-(6-((2-(2-oxoindolin-1-yl)ethyl)amino)pyrimidin-4-yl)benzoate

The title compound is prepared according to the general procedure A described above using 1-(2-((6-chloropyrimidin-4-yl)amino)ethyl)indolin-2-one (example 4-b) and 4-ethoxycarbonylphenylboronic acid; LC-MS A: t_{R} = 0.69 min; [M+H]⁺ = 402.94.

### Biological in vitro Assays

The antagonistic activities of the compounds of formula (I) on the EP2 and EP4 receptors are determined in accordance with the following experimental methods.

The assay is using the PathHunterTM HEK 293 PTGER2 and PTGER4 b-arrestin cell lines from DiscoverX. The system is based on the Enzyme Fragment Complementation Technology. Two complementing fragments of the b-galactosidase enzyme are expressed within stably transfected cells. The larger portion of b-gal, termed EA for Enzyme Acceptor, is fused to the C-terminus of b-arrestin 2. The smaller fragment, termed ProLinkTM tag, is fused to PTGER2 (EP2) or PTRGER4 (EP4) at the C-terminus. Upon activation, b-arrestin is recruited which forces the interaction of ProLink and EA, allowing complementation of the two fragments of b-gal and the formation of a functional enzyme which is capable of hydrolysing the substrate and generating a chemiluminescent signal.

### hEP2 b-arrestin assay:

The HEK 293 PTGER2 b-arrestin cells (DiscoverX 93-021-4C1) are detached from culture dishes with a cell dissociation buffer (Invitrogen, 13151-014), and collected in growing medium (GM: DMEM + Glutamax-I (Invitrogen 32430) /10% FCS, 1 % Penicilin/streptomycin). 5000 cells per well of a 384 well plate (white with white bottom Greiner 781080) are seeded in 20ul per well of GM. Plate is incubated at 37°C, 5% CO2 for 24 hours.

Stock solutions of test compounds are made at a concentration of 10 mM in DMSO, and serially diluted in DMSO to concentrations required for inhibition dose response curves (tested concentration range 10µM-2nM or 1µM-0.2nM).

PGE2 (Cayman 14010, stock solution: 10mM in DMSO) is used as agonist at 5µM final concentration, corresponding to EC80.

Five microliters of diluted compounds are transferred into the assay plate. Plate is pre-incubated 15 minutes at 37°C. Then five microliters of PGE2 (final conc. 5µM) are transferred into the assay plate. Plate is incubated 120 minutes at 37°C.

PathHunter Glo Detection Kit components are thawed and mix according to manufacturer's instructions : 1 part Galacton Star Substrate with 5 parts Emerald IITM Solution, and 19 parts of PathHunter Cell Assay Buffer, respectively. Twelve µL of reagent are transferred to the assay plate and incubate for 1 hour at room temperature in the dark. Luminescence counts are read on a BMG Fluostar Optima reader according to manufacturer's instructions.

For each compound concentration calculate of the percentage of activity compared to DMSO control value as average ± STDEV. (each concentration is measured in duplicate)
IC50 values and curves are generated with XLfit software (IDBS) using Dose-Response One Site model 203. When compounds were measured multiple times, mean values are given.

### hEP4 b-arrestin assay:

The HEK 293 PTGER4 b-arrestin cells (DiscoverX 93-030-4C1) are detached from culture dishes with a cell dissociation buffer (Invitrogen, 13151-014), and collected in growing medium (GM: DMEM + Glutamax-I (Invitrogen 32430) /10% FCS, 1 % Penicilin/streptomycin). 5000 cells per well of a 384 well plate (white with white bottom Greiner 781080) are seeded in 20µl per well of GM. Plate is incubated at 37°C, 5% CO2 for 24 hours.

Stock solutions of test compounds are made at a concentration of 10 mM in DMSO, and serially diluted in DMSO to concentrations required for inhibition dose response curves (tested concentration range 10µM-2nM or 1µM-0.2nM).

PGE2 (Cayman 14010, stock solution: 100uM in DMSO) is used as agonist at 20nM final concentration, corresponding to EC80.

Five microliters of diluted compounds are transferred into the assay plate. Plate is pre-incubated 15 minutes at 37°C. Then five microliters of PGE2 (final conc. 20nM) are transferred into the assay plate. Plate is incubated 120 minutes at 37°C.

PathHunter Glo Detection Kit components are thawed and mix according to manufacturer's instructions : 1 part Galacton Star Substrate with 5 parts Emerald IITM Solution, and 19 parts of PathHunter Cell Assay Buffer, respectively. Twelve 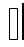 l of reagent are transferred to the assay plate and incubate for 1 hour at room temperature in the dark. Luminescence counts are read on a BMG Fluostar Optima reader according to manufacturer's instructions.

For each compound concentration calculate of the percentage of activity compared to DMSO control value as average ± STDEV. (each concentration is measured in duplicate)
IC50 values and curves are generated with XLfit software (IDBS) using Dose-Response One Site model 203. When compounds were measured multiple times, mean values are given.

The antagonistic activities of the compounds of formula (I) on the EP2 and EP4 receptors are also determined in accordance with the following experimental method.

Human tumor cell lines expressing endogenously either EP4 or EP2 are used and cAMP accumulation in cells upon PGE2 stimulation is monitored. SF295 glioblastoma cells express high endogenous EP2 and no EP4,whereas BT549 breast cancer cells, express high endogenous EP4 levels and very low EP2 levels.

As a detection method for cAMP the HTRF (homogeneous time resolved fluorescence) Cisbio kit (HTRF cAMP dynamic 2 kit 20'000 tests Cisbio Cat. #62AM4PEC) was used, which is based on a competitive immunoassay using a Cryptate-labeled anti-cAMP antibody and d2-labeled cAMP. Native cAMP produced by cells or unlabeled cAMP (for the standard curve) compete with exogenously added d2-labeled cAMP (acceptor) for binding to monoclonal anti-cAMP-Eu3+ Cryptate (donor). A FRET signal (Fluorescence Resonance Energy Transfer) is obtained only if the labeled anti-cAMP antibody binds the d2 labelled cAMP, thus the specific signal (i.e. energy transfer) is inversely proportional to the concentration of cAMP in the standard or sample.

### hEP2 cAMP assay:

The SF295 cells (NCI/No. 0503170) are detached from culture dishes with a cell dissociation buffer (Invitrogen, 13151-014), and collected in growing medium (GM: RPMI1640 (Invitrogen 21875) /10% FCS, 1 % Penicilin/streptomycin). Cells are counted washed and resuspended in assay buffer (AB; HBSS, 20mM HEPES, 0.2% BSA; 2mM IBMX). 4'000 cells in 5 µL of AB are seeded per well of a small volume 384 well plate (black with flat bottom, Greiner 784076).

Stock solutions of test compounds are made at a concentration of 10 mM in DMSO, and serially diluted in DMSO to concentrations required for inhibition dose response curves (tested concentration range 30µM - 0.4nM; 30 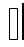 M - 0.015nM or 1µM -0.01nM).

PGE2 (Cayman 14010, stock solution: 75µM in DMSO) is used as agonist at 75nM final concentration, corresponding to EC80.

2.5 µL of diluted compounds are transferred into the assay plate. Plate is pre-incubated 45 minutes at room temperature. Subsequently, 2.5 µL of PGE2 (final conc. 75nM) are transferred into the assay plate. Plate is incubated 30 minutes at room temperature. Five 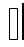 l of each donor (anti-cAMP cryptate) and acceptor (cAMP-d2) are added and the plate is incubated another hour at room temperature in the dark and then read using a BMG LABTECH PHERAstar reader (Excitation : 337nm, Emission : 620 and 665nm).

The obtained Delta F (fluorescence) values (665nm/620nM) are converted into % cAMP values using the measurements of the cAMP calibrator provided in the kit. For each compound concentration the percentage of cAMP compared to DMSO control value as average ± STDEV (each concentration is measured in duplicate) is calculated.

IC50 values and curves are generated with XLfit software (IDBS) using Dose-Response One Site model 203. When compounds were measured multiple times, mean values are given.

### hEP4 cAMP assay:

The BT549 cells (NCI/No. 0507282) are detached from culture dishes with a cell dissociation buffer (Invitrogen, 13151-014), and collected in growing medium (GM: RPMI1640 (Invitrogen 21875) /10% FCS, 1 % Penicilin/streptomycin). Cells are counted washed and resuspended in assay buffer (AB; HBSS, 20mM HEPES, 0.2% BSA; 2mM IBMX). 4'000 cells in 5 µL of AB are seeded per well of a small volume 384 well plate (black with flat bottom, Greiner 784076).

Stock solutions of test compounds are made at a concentration of 10 mM in DMSO, and serially diluted in DMSO to concentrations required for inhibition dose response curves (tested concentration range 30µM - 0.4nM; 30µM - 0.015nM or 1µM - 0.01nM).

PGE2 (Cayman 14010, stock solution: 6µM in DMSO) is used as agonist at 6nM final concentration, corresponding to EC80.

2.5 µL of diluted compounds are transferred into the assay plate. Plate is pre-incubated 45 minutes at room temperature. Subsequently, 2.5 µL of PGE2 (final conc. 6nM) are transferred into the assay plate. Plate is incubated 30 minutes at room temperature. Five µL of each donor (anti-cAMP cryptate) and acceptor (cAMP-d2) are added and the plate is incubated another hour at room temperature in the dark and then read using a BMG LABTECH PHERAstar reader (Excitation : 337nm, Emission : 620 and 665nm).

The obtained Delta F (fluorescence) values (665nm/620nM) are converted into % cAMP values using the measurements of the cAMP calibrator provided in the kit. For each compound concentration the percentage of cAMP compared to DMSO control value as average ± STDEV (each concentration is measured in duplicate) is calculated.

IC50 values and curves are generated with XLfit software (IDBS) using Dose-Response One Site model 203. When compounds were measured multiple times, mean values are given.

Antagonistic activities of exemplified compounds are displayed in **Table 3:**

**Table 3:**

| **Ex** | hEP2 beta-arrestin IC50 [nM] | hEP2 cAMP IC50 (nM) | hEP4 beta-arrestin IC50 (nM) | hEP4 cAMP IC50 (nM) |
|---|---|---|---|---|
| **1** | 13 | 15 | 5970 | 5718 |
| **2** | 11 | 10 | 2059 | 1350 |
| **3** | 8 | 5 | 11020 | 5660 |
| **4** | 3 | 2 | 2965 | 2615 |
| **5** | 17 | | 4973 | |

## Claims

1. A compound of formula (I) 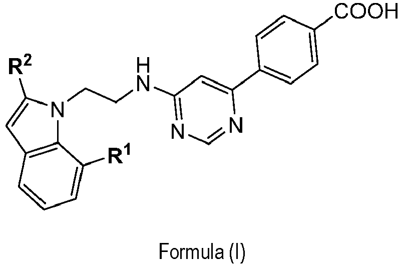 wherein
**R¹** represents hydrogen or methyl;
**R²** represents methyl, bromo, chloro, or cyano;
or a pharmaceutically acceptable salt thereof.

2. A compound according to claim 1; wherein **R¹** represents hydrogen;
or a pharmaceutically acceptable salt thereof.

3. A compound according to claim1; wherein **R¹** represents methyl;
or a pharmaceutically acceptable salt thereof.

4. A compound according to any one of claims 1 to 3; wherein **R²** represents methyl;
or a pharmaceutically acceptable salt thereof.

5. A compound according to any one of claims 1 to 3; wherein **R²** represents chloro;
or a pharmaceutically acceptable salt thereof.

6. A compound according to any one of claims 1 to 3; wherein **R²** represents cyano;
or a pharmaceutically acceptable salt thereof.

7. A compound according to claim 1 selected from the group consisting of:
4-{6-[2-(2-Methyl-indol-1-yl)-ethylamino]-pyrimidin-4-yl}-benzoic acid;
4-{6-[2-(2-Cyano-indol-1-yl)-ethylamino]-pyrimidin-4-yl}-benzoic acid;
4-{6-[2-(2,7-Dimethyl-indol-1-yl)-ethylamino]-pyrimidin-4-yl}-benzoic acid;
4-{6-[2-(2-Chloro-indol-1-yl)-ethylamino]-pyrimidin-4-yl}-benzoic acid; and
4-{6-[2-(2-Bromo-indol-1-yl)-ethylamino]-pyrimidin-4-yl}-benzoic acid;
or a pharmaceutically acceptable salt thereof.

8. A pharmaceutical composition comprising, as active principle, a compound according to any one of claims 1 to 7, or a pharmaceutically acceptable salt thereof, and at least one therapeutically inert excipient.

9. A compound according to any one of claims 1 to 7, or a pharmaceutically acceptable salt thereof, for use as a medicament.

10. A compound according to any one of claims 1 to 7, or a pharmaceutically acceptable salt thereof, for use in the prevention or treatment of diseases selected from the group consisting of cancer; pain; endometriosis; autosomal dominant polycystic kidney disease; acute ischemic syndromes in atherosclerotic patients; pneumonia; and neurodegenerative diseases; or for use in the control of female fertility.

11. A compound according to any one of claims 1 to 7, or a pharmaceutically acceptable salt thereof, for use in the prevention or treatment of a cancer selected from melanoma; lung cancer; bladder cancer; renal carcinomas; gastro-intestinal cancers; endometrial cancer; ovarian cancer; cervical cancer; and neuroblastoma.

12. A compound according to any one of claims 1 to 7, or a pharmaceutically acceptable salt thereof, for use in the preparation of a medicament for the prevention or treatment of diseases selected from the group consisting of cancer; pain; endometriosis; autosomal dominant polycystic kidney disease; acute ischemic syndromes in atherosclerotic patients; pneumonia; and neurodegenerative diseases; or for the control of female fertility.

13. A compound according to any one of claims 1 to 7, or a pharmaceutically acceptable salt thereof, for use in a method of modulating an immune response in a subject having a tumor; wherein said method reactivates the immune system in the tumor of said subject.

14. A compound according to any one of claims 1 to 7, or a pharmaceutically acceptable salt thereof, for use in the prevention or treatment of cancer; wherein said compound is used in combination with a modulator of the PGE2 receptor EP4; and, in addition, optionally in combination with one or more chemotherapy agents and / or radiotherapy and / or targeted therapy.

## Patentansprüche

1. Verbindung von Formel (I) 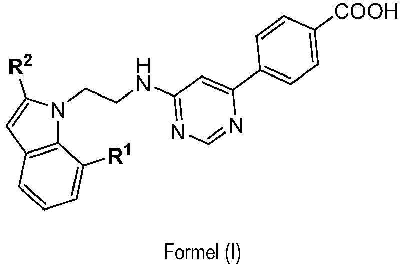 wobei
**R¹** Wasserstoff oder Methyl repräsentiert;
**R²** Methyl, Brom, Chlor oder Cyano repräsentiert;
oder ein pharmazeutisch akzeptables Salz davon.

2. Verbindung nach Anspruch 1, wobei **R¹** Wasserstoff repräsentiert;
oder ein pharmazeutisch akzeptables Salz davon.

3. Verbindung nach Anspruch 1, wobei **R¹** Methyl repräsentiert;
oder ein pharmazeutisch akzeptables Salz davon.

4. Verbindung nach einem der Ansprüche 1 bis 3, wobei **R²** Methyl repräsentiert;
oder ein pharmazeutisch akzeptables Salz davon.

5. Verbindung nach einem der Ansprüche 1 bis 3, wobei **R²** Chlor repräsentiert;
oder ein pharmazeutisch akzeptables Salz davon.

6. Verbindung nach einem der Ansprüche 1 bis 3, wobei **R²** Cyano repräsentiert;
oder ein pharmazeutisch akzeptables Salz davon.

7. Verbindung nach Anspruch 1, ausgewählt aus der Gruppe bestehend aus:
4-{6-[2-(2-Methyl-indol-1-yl)-ethylamino]-pyrimidin-4-yl}-benzoesäure;
4-{6-[2-(2-Cyano-indol-1-yl)-ethylamino]-pyrimidin-4-yl}-benzoesäure:
4-{6-[2-(2,7-Dimethyl-indol-1-yl)-ethylamino]-pyrimidin-4-yl}-benzoesäure;
4-{6-[2-(2-Chlor-indol-1-yl)-ethylamino]-pyrimidin-4-yl}-benzoesäure; und
4-{6-[2-(2-Brom-indol-1-yl)-ethylamino]-pyrimidin-4-yl}-benzoesäure;
oder ein pharmazeutisch akzeptables Salz davon.

8. Pharmazeutische Zusammensetzung, die als Wirkstoff eine Verbindung nach einem der Ansprüche 1 bis 7
oder ein pharmazeutisch akzeptables Salz davon und wenigstens einen therapeutisch inerten Hilfsstoff umfasst.

9. Verbindung nach einem der Ansprüche 1 bis 7 oder ein pharmazeutisch akzeptables Salz davon zur Verwendung als Medikament.

10. Verbindung nach einem der Ansprüche 1 bis 7 oder ein pharmazeutisch akzeptables Salz davon zur Verwendung bei der Verhütung oder Behandlung von Krankheiten, ausgewählt aus der Gruppe bestehend aus Krebs; Schmerz; Endometriose; autosomal-dominanter polyzystischer Nierenerkrankung; akuten ischämischen Syndromen bei Patienten mit Atherosklerose; Lungenentzündung; und neurodegenerativen Krankheiten; oder zur Verwendung bei der Kontrolle weiblicher Fruchtbarkeit.

11. Verbindung nach einem der Ansprüche 1 bis 7 oder ein pharmazeutisch akzeptables Salz davon zur Verwendung bei der Verhütung oder Behandlung einer Krebserkrankung, ausgewählt aus Melanom; Lungenkrebs; Blasenkrebs; Nierenkarzinomen; Magen-Darm-Krebs; Endometriumkrebs; Eierstockkrebs; Gebärmutterhalskrebs; und Neuroblastom.

12. Verbindung nach einem der Ansprüche 1 bis 7 oder ein pharmazeutisch akzeptables Salz davon zur Verwendung bei der Herstellung eines Medikaments zur Verhütung oder Behandlung von Krankheiten, ausgewählt aus der Gruppe bestehend aus Krebs; Schmerz; Endometriose; autosomal-dominanter polyzystischer Nierenerkrankung; akuten ischämischen Syndromen bei Patienten mit Atherosklerose; Lungenentzündung; und neurodegenerativen Krankheiten; oder zur Kontrolle weiblicher Fruchtbarkeit.

13. Verbindung nach einem der Ansprüche 1 bis 7 oder ein pharmazeutisch akzeptables Salz davon zur Verwendung in einem Verfahren zur Modulation einer Immunreaktion in einem Subjekt mit einem Tumor, wobei das genannte Verfahren das Immunsystem im Tumor des genannten Subjekts reaktiviert.

14. Verbindung nach einem der Ansprüche 1 bis 7 oder ein pharmazeutisch akzeptables Salz davon zur Verwendung bei der Verhütung oder Behandlung von Krebs, wobei die genannte Verbindung in Kombination mit einem Modulator des PGE2-Rezeptors EP4 und zusätzlich optional in Kombination mit einem oder mehreren Chemotherapeutika und/oder Strahlentherapie und/oder gezielter Therapie verwendet wird.

## Revendications

1. Composé de formule (I) 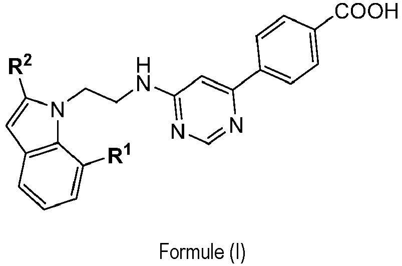 où
**R¹** représente un hydrogène ou un méthyle ;
**R²** représente un méthyle, un bromo, un chloro ou un cyano ;
ou sel pharmaceutiquement acceptable de celui-ci.

2. Composé selon la revendication 1 ; où **R¹** représente un hydrogène ;
ou sel pharmaceutiquement acceptable de celui-ci.

3. Composé selon la revendication 1 ; où **R¹** représente un méthyle ;
ou sel pharmaceutiquement acceptable de celui-ci.

4. Composé selon l'une quelconque des revendications 1 à 3 ; où **R²** représente un méthyle ;
ou sel pharmaceutiquement acceptable de celui-ci.

5. Composé selon l'une quelconque des revendications 1 à 3 ; où **R²** représente un chloro ;
ou sel pharmaceutiquement acceptable de celui-ci.

6. Composé selon l'une quelconque des revendications 1 à 3 ; où **R²** représente un cyano ;
ou sel pharmaceutiquement acceptable de celui-ci.

7. Composé selon la revendication 1 sélectionné dans le groupe consistant en les suivants :
Acide 4-{6-[2-(2-méthyl-indol-1-yl)-éthylamino]-pyrimidin-4-yl}-benzoïque ;
Acide 4-{6-[2-(2-cyano-indol-1-yl)-éthylamino]-pyrimidin-4-yl}-benzoïque ;
Acide 4-{6-[2-(2,7-diméthyl-indol-1-yl)-éthylamino]-pyrimidin-4-yl}-benzoïque ;
Acide 4-{6-[2-(2-chloro-indol-1-yl)-éthylamino]-pyrimidin-4-yl}-benzoïque ; et
Acide 4-{6-[2-(2-bromo-indol-1-yl)-éthylamino]-pyrimidin-4-yl}-benzoïque ;
ou sel pharmaceutiquement acceptable de celui-ci.

8. Composition pharmaceutique comprenant, comme principe actif, un composé selon l'une quelconque des revendications 1 à 7 ou un sel pharmaceutiquement acceptable de celui-ci et au moins un excipient thérapeutiquement inerte.

9. Composé selon l'une quelconque des revendications 1 à 7 ou sel pharmaceutiquement acceptable de celui-ci pour une utilisation en tant que médicament.

10. Composé selon l'une quelconque des revendications 1 à 7 ou sel pharmaceutiquement acceptable de celui-ci pour une utilisation dans la prévention ou le traitement de maladies sélectionnées dans le groupe consistant en un cancer ; la douleur ; l'endométriose ; la polykystose rénale autosomique dominante ; les syndromes ischémiques aigus chez les patients athéroscléreux ; la pneumonie ; et des maladies neurodégénératives ; ou pour une utilisation dans le contrôle de la fécondité féminine.

11. Composé selon l'une quelconque des revendications 1 à 7 ou sel pharmaceutiquement acceptable de celui-ci pour une utilisation dans la prévention ou le traitement d'un cancer sélectionné parmi un mélanome ; un cancer du poumon ; un cancer de la vessie ; des carcinomes rénaux ; des cancers gastro-intestinaux ; un cancer de l'endomètre ; un cancer de l'ovaire ; un cancer du col de l'utérus ; et un neuroblastome.

12. Composé selon l'une quelconque des revendications 1 à 7 ou sel pharmaceutiquement acceptable de celui-ci pour une utilisation dans la préparation d'un médicament pour la prévention ou le traitement de maladies sélectionnées dans le groupe consistant en un cancer ; la douleur ; l'endométriose ; la polykystose rénale autosomique dominante ; les syndromes ischémiques aigus chez les patients athéroscléreux ; la pneumonie ; et des maladies neurodégénératives ; ou pour le contrôle de la fécondité féminine.

13. Composé selon l'une quelconque des revendications 1 à 7 ou sel pharmaceutiquement acceptable de celui-ci pour une utilisation dans une méthode de modulation de la réponse immunitaire chez un sujet qui présente une tumeur ; où ladite méthode conduit à une réactivation de la réponse immunitaire dans la tumeur dudit sujet.

14. Composé selon l'une quelconque des revendications 1 à 7 ou sel pharmaceutiquement acceptable de celui-ci pour une utilisation dans la prévention ou le traitement d'un cancer ; où ledit composé est utilisé en combinaison avec un modulateur du récepteur EP4 à la PGE2 ; et en outre éventuellement en combinaison avec un ou plusieurs agents chimiothérapeutiques et/ou une radiothérapie et/ou une thérapie ciblée.
